(19) Europäisches Patentamt
European Patent Office
Office européen des brevets

(11) **EP 2 815 772 A1**

(12) **EUROPÄISCHE PATENTANMELDUNG**

(43) Veröffentlichungstag:
**24.12.2014 Patentblatt 2014/52**

(51) Int Cl.:
*A61L 26/00* *(2006.01)*       *A61L 24/00* *(2006.01)*
*A61L 24/06* *(2006.01)*       *A61L 24/08* *(2006.01)*
*A61L 24/10* *(2006.01)*

(21) Anmeldenummer: **14172496.3**

(22) Anmeldetag: **16.06.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **17.06.2013   DE 102013211316**

(71) Anmelder: **AESCULAP AG**
**78532 Tuttlingen (DE)**

(72) Erfinder:
• **Bargon, Rainer**
  **78532 Tuttlingen (DE)**
• **Odermatt, Erich**
  **8200 Schaffhausen (CH)**

(74) Vertreter: **Patentanwälte**
**Ruff, Wilhelm, Beier, Dauster & Partner**
**Kronenstraße 30**
**70174 Stuttgart (DE)**

(54) **Hämostyptikum**

(57)    Die Erfindung betrifft ein Hämostyptikum, umfassend ein hämostyptisch wirksames Material und ein Visualisierungsmittel.

Außerdem betrifft die Erfindung ein Kit sowie eine Austragsvorrichtung, welche jeweils das Hämostyptikum umfassen.

Fig. 1

## Beschreibung

Die Erfindung betrifft ein Hämostyptikum sowie ein Kit und eine Austragsvorrichtung, welche jeweils das Hämostyptikum umfassen.

Chirurgische Operationen im minimalinvasiven Bereich werden grundsätzlich von intraoperativ auftretenden Blutungen begleitet. Derartige Blutungen gehen mit zahlreichen Risiken einher. Sie können beispielsweise zu einem eingeschränkten Sichtfeld des Chirurgen führen, wodurch die Präzision eines chirurgischen Eingriffs beeinträchtigt wird.

Konventionelle Methoden zur Stillung von intraoperativ auftretenden Blutungen sehen beispielsweise die Verwendung von elektrischer Koagulation, Nahtmaterialien zur Ligatur oder Staplern vor. Diese Methoden sind jeweils traumatischer Natur und verursachen in der Regel Gewebereizungen.

Ein weiteres Problem von intraoperativen Blutungen stellt die Ausbildung von postoperativen Gewebeadhäsionen durch die Bildung von Fibrinsträngen zwischen anliegenden Organoberflächen dar.

Weiterhin können intraoperativ auftretende Blutungen die Einschleppung von Keimen und infolgedessen das Auftreten von Spätinfektionen begünstigen.

Eine weitere Gefahr, die mit intraoperativ auftretenden Blutungen einhergeht, besteht in der Ausbildung von Thrombosen als Folge einer unkontrollierten Blutgerinnung.

Schließlich können intraoperativ auftretende Blutungen während oder nach einer Operation zu einem Blutdruckabfall und einer Destabilisierung des Patientenkreislaufs führen.

Von den oben genannten Risiken stellt insbesondere die Infektionsgefahr durch Kontakt mit kontaminiertem Operationsbesteck oder aerogenen Pathogenen ein Problem bei der nachfolgenden Wundheilung dar. Letztere kann bekanntermaßen in eine inflammatorische, exsudative und proliferative Phase unterteilt werden. Alle drei Phasen der Wundheilung werden unter dem Einfluss von Pathogenen deutlich verlängert, so dass anstelle einer primären Wundheilung eine sekundäre Wundheilung mit einer ausgeprägten Entzündungsphase auftreten kann.

Eine modifizierte Stärke in Form eines Schwamms, Schaums oder Pulvers zur Blutstillung ist aus der CN 101497670 A bekannt.

In der WO 2011/031457 A2 ist ein Antiadhäsionsmittel auf Basis einer Polymerkombination aus einem biodegradierbaren Polymer, beispielsweise einem Copolymer aus Lactid und Glykolid, und einem wasserlöslichen Polymer, beispielsweise einem Polyvinylalkohol oder einem Polyethylenglykol, beschrieben.

Gegenstand der WO 2009/091549 A1 ist eine bioverträgliche, modifizierte Stärke, welche unter anderem zur Blutstillung vorgesehen ist.

Die WO 2005/002510 A2 betrifft ein blutstillendes Material, welches als blutstillendes Substrat Chitosan enthält.

Ein zur Blutstillung verwendbarer modifizierter Stärkeschwamm bzw. -schaum ist aus der EP 2 233 157 A1 bekannt.

Eine blutstillende Aerosolzusammensetzung, welche mikrodispergierte Polyanhydroglucuronsäure sowie ein Treibgas enthält, ist Gegenstand der WO 98/33479 A1.

Die Verwendung von porösen Teilchen zur Herstellung eines Medikaments zur Behandlung zum Steigern der Erzeugung von Gerinnungen ist aus der EP 1 025 868 B1 bekannt.

Medikamente mit über intra- und/oder intermolekularen Esterbindungen quervernetzten, Carboxylgruppen tragenden Polysacchariden sind in der US 5,676,964 beschrieben.

Aus der CN 1269376 A ist die Herstellung eines antiadhäsiven Produkts unter Verwendung eines Cellulosematerials bekannt.

Beispiele für kommerziell erhältliche Hämostyptika sind die unter den Bezeichnungen HaemoCer™, PerClot® sowie Arista® AH vertriebenen pulverförmigen Produkte.

Obgleich konventionelle Hämostyptika bzw. blutstillende Mittel den grundsätzlichen Anforderungen für eine erfolgreiche Blutstillung genügen, ist ihr Einsatz in der Regel auf die offene Chirurgie und/oder auf gut zugängliche Stellen in einem Behandlungssitus begrenzt. Zudem kann eine Kontrolle der Effizienz auch durch einen Überschuss an hämostyptischem Material erschwert werden, indem beispielsweise zweidimensional ausgestaltete Hämostyptika, insbesondere in Form von Vliesen, eingesetzt werden.

## Aufgabe und Lösung

Der vorliegenden Erfindung lag daher die Aufgabe zugrunde, ein Hämostyptikum, ein Kit sowie eine Austragsvorrichtung bereitzustellen, welche insbesondere die einleitend erwähnten Nachteile vermeiden und obendrein eine einfache und zuverlässige Überprüfung der hämostyptischen Wirkung während oder nach einer medizinischen Behandlung ermöglichen.

Diese Aufgabe wird in einem ersten Erfindungsaspekt gelöst durch ein Hämostyptikum gemäß unabhängigem Anspruch 1. In einem zweiten Aspekt wird die der Erfindung zugrundeliegende Aufgabe durch ein Kit mit den Merkmalen des Anspruchs 15 gelöst. In einem dritten Aspekt wird die der Erfindung zugrundeliegende Aufgabe durch eine Austragsvorrichtung mit den Merkmalen des Anspruchs 16 gelöst. Bevorzugte Ausführungsformen des Hämostyptikums sind in den abhängigen Ansprüchen 2 bis 14 definiert. Der Gegenstand sämtlicher Ansprüche wird hiermit durch ausdrückliche Bezugnahme zum Inhalt der vorliegenden Beschreibung gemacht.

Die Erfindung schlägt in einem ersten Aspekt ein Hämostyptikum, d.h. ein blutstillendes Mittel, vor, welches ein hämostyptisch wirksames Material und ein Visualisierungsmittel umfasst.

Hierdurch sind die folgenden Vorteile erzielbar:

➢ Mittels des Visualisierungsmittels ist es möglich, eine sichere und zuverlässige Überprüfung der Wirksamkeit des Hämostyptikums vorzunehmen, und zwar sowohl während als auch nach einem medizinischen, insbesondere chirurgischen, Eingriff.

➢ Dies ist vor allem bei der hämostyptischen bzw. blutstillenden Behandlung von schwer zugänglichen und/oder einsehbaren Körperregionen sowie in den Fällen von Vorteil, in denen das Hämostyptikum vollständig mit Blut bedeckt ist, was insbesondere bei stark blutenden Behandlungssitus, insbesondere Wundsitus, regelmäßig der Fall ist.

➢ Hierdurch ist mit besonderem Vorteil eine Reduktion von unnötigem Fremdmaterial möglich.

➢ Schließlich ist im Falle eines pulverförmigen Hämostyptikums von Vorteil, dass dieses mittels Ausspülen aus einem Behandlungssitus, insbesondere Wundsitus, nach erfolgter Blutstillung entfernt werden kann, der Erfolg der Blutstillung unmittelbar sichtbar wird und keine Partikel in Gefäße gelangen können, womit iatrogenen Thrombosen vorgebeugt werden kann.

Der Ausdruck "hämostyptisch wirksames Material" bedeutet im Sinne der vorliegenden Erfindung ein Material, welches in der Lage ist, eine Blutstillung bzw. Hämostase zu bewirken.

Der Ausdruck "ein hämostyptisch wirksames Material" kann im Sinne der vorliegenden Erfindung ein hämostyptisch wirksames Material (Singular) oder eine Mehrzahl (Plural), d.h. zwei oder mehr, hämostyptisch wirksame Materialien bzw. eine Mischung von verschiedenen hämostyptisch wirksamen Materialien bedeuten. Auf geeignete hämostyptisch wirksame Materialien wird im Folgenden noch näher eingegangen werden.

Der Ausdruck "Visualisierungsmittel" bedeutet im Sinne der vorliegenden Erfindung ein Mittel, welches dazu ausgebildet ist, eine Visualisierung bzw. Sichtbarmachung des Hämostyptikums und/oder einer Blutstillung zu bewirken. Die Visualisierung des Hämostyptikums beruht vorzugsweise auf der Erzeugung eines optischen, insbesondere farbigen, Kontrasts gegenüber Blut bzw. einer bluthaltigen Körperflüssigkeit. Das erfindungsgemäß vorgesehene Visualisierungsmittel eignet sich somit insbesondere als Hämostasemarker und/oder als Marker zur Auffindung von überschüssigem Hämostyptikum im Blut eines Patienten.

Der Ausdruck "ein Visualisierungsmittel" kann im Sinne der vorliegenden Erfindung ein Visualisierungsmittel (Singular) oder eine Mehrzahl (Plural), d.h. zwei oder mehr, Visualisierungsmittel bzw. eine Mischung von verschiedenen Visualisierungsmitteln bedeuten. Auf geeignete Visualisierungsmittel wird im Folgenden noch näher eingegangen werden.

Der Ausdruck "Behandlungssitus" bedeutet im Sinne der vorliegenden Erfindung ein unter medizinischen Gesichtspunkten behandlungswürdiges Körperareal. In der Regel handelt es sich hierbei um eine Wunde, welche durch einen operativen Eingriff oder durch andere traumatische Ereignisse oder durch Erkrankungen bedingt sein kann. Entsprechend kann es sich bei dem Behandlungssitus um einen Wund- bzw. Operationssitus handeln.

Das Hämostyptikum liegt in einer bevorzugten Ausführungsform in Form einer das hämostyptisch wirksame Material und das Visualisierungsmittel enthaltenden Mischung vor.

Insbesondere kann das hämostyptisch wirksame Material mit dem Visualisierungsmittel versehen bzw. additiviert sein.

Grundsätzlich können das hämostyptisch wirksame Material und das Visualisierungsmittel kovalent miteinander verbunden sein.

Erfindungsgemäß ist es indes bevorzugt, wenn das hämostyptisch wirksame Material und das Visualisierungsmittel nichtkovalent, beispielsweise mittels adsorptiven Kräften, Van-der-Waals-Kräften und/oder Wasserstoffbrückenbindungen, miteinander verbunden sind.

In einer besonders bevorzugten Ausführungsform liegt das Hämostyptikum, insbesondere das hämostyptisch wirksame Material und/oder das Visualisierungsmittel, in Form von Partikeln vor.

Beispielsweise kann das Hämostyptikum, insbesondere das hämostyptisch wirksame Material und/oder das Visualisierungsmittel, als Puder, Pulver oder Granulat vorliegen.

Das Hämostyptikum, insbesondere das hämostyptisch wirksame Material und/oder das Visualisierungsmittel, weist in einer weitergehenden Ausführungsform eine Partikelgröße kleiner 200 $\mu$m, insbesondere eine Partikelgröße

von 1 μm bis 60 μm, auf. Die in diesem Absatz genannten Partikelgrößen haben insbesondere den Vorteil, dass sie die Austragsöffnungen, insbesondere Austrittsdüsen, von herkömmlichen Austragsvorrichtungen, insbesondere Spraydosen, ohne größere Schwierigkeiten und insbesondere ohne Verursachung von Verstopfungen passieren können. Ein weiterer Vorteil besagter Partikelgrößen besteht darin, dass die Partikel - auch nach längerer Lagerung an Luft - im Wesentlichen trocken vorliegen. Der Ausdruck "im Wesentlichen trocken" bedeutet an dieser Stelle, dass die Partikel - abhängig von der vorherrschenden Raumluftfeuchte - einen Wassergehalt von 1 ppm bis 10000 ppm aufweisen können.

In einer vorteilhaften Ausführungsform bildet das Hämostyptikum keine Dispersionen, insbesondere keine wässerigen Dispersionen, hoher Viskosität. Bevorzugt bildet das Hämostyptikum Dispersionen, insbesondere wässerige Dispersionen, mit einer komplexen Viskosität kleiner 40 Pas, insbesondere mit einer komplexen Viskosität von 20 Pas bis 30 Pas (Bohlin Gemini Messsystem mit Kegel-Platte-System 4°/40, isotherm bei 37°C, 900 s Ausgleichszeit, 5 Hz, Deformation 0.003, ohne stationäre Vorscherung im Oszillationsmodus). Dadurch lässt sich das Hämostyptikum im Bedarfsfall mühelos wieder von einem Behandlungssitus, beispielsweise mittels Spülen unter Zuhilfenahme einer Kochsalzlösung, entfernen. Unter dem Ausdruck "komplexe Viskosität" soll im Sinne der vorliegenden Erfindung der Summenparameter aus Elastizitätsmodul (bzw. Speichermodul) und Verlustmodul verstanden werden. Das Elastizitäts- bzw. Speichermodul spiegelt die gespeicherte mechanische Energie wider. Das Verlustmodul spiegelt den dissipativ, d.h. unwiederbringlich freigesetzten, viskosen Anteil der in die Dispersion eingebrachten Energie wider. Die komplexe Viskosität wird in der Regel mittels der sogenannten Oszillationsmessung ermittelt. Die Oszillationsmessung zur Ermittlung der komplexen Viskosität eines Materials ist dem Fachmann an sich bekannt, so dass an dieser Stelle auf weiterführende Erläuterungen verzichtet wird (Thomas Mezger: Das Rheologie-Handbuch: Für Anwender von Rotations- und Oszillations-Rheometern, 3. Auflage, Curt R. Vincentz Verlag, Hannover: 2010, 441 Seiten).

Das hämostyptisch wirksame Material ist in einer weiteren Ausführungsform ein mucoadhäsives Material. Unter dem Ausdruck "mucoadhäsives Material" soll im Sinne der vorliegenden Erfindung ein Material verstanden werden, das in der Lage ist, Wasser in Form von Hydrathüllen zu binden und eine adhäsive Bindung zur Mucinschicht von Schleimhäuten einzugehen. Die Mucinschicht besteht grundsätzlich aus Glykoproteinen (Mucine), die Sulfat- und Carboxylgruppen aufweisen und aufgrund dessen große Mengen an Wasser in Form von Hydrathüllen binden können. Die Mucoadhäsion beruht vorzugsweise auf ionischen Wechselwirkungen und/oder der Ausbildung von Wasserstoffbrückenbindungen zwischen dem mucoadhäsiven Material und den Glykoproteinen und insbesondere Calciumionen der Mucinschicht des Gewebes. Darüber hinaus kann im Falle eines langkettigen Polymermaterials eine Verschlaufung des Materials mit den Polymerketten der Glykoproteine stattfinden, was ebenfalls eine Mucoadhäsion zur Folge hat.

Mit anderen Worten kann es daher erfindungsgemäß vorgesehen sein, dass das hämostyptisch wirksame Material in der Lage ist, über ionische Wechselwirkungen und/oder die Ausbildung von Wasserstoffbrückenbindungen an biologisches, insbesondere menschliches und/oder tierisches, Gewebe zu binden.

Das hämostyptisch wirksame Material kann insbesondere funktionelle Gruppen aufweisen, welche ausgewählt sind aus der Gruppe umfassend Carboxylgruppen, Sulfatgruppen, Phosphatgruppen und Kombinationen davon. Die in diesem Absatz beschriebenen Ausführungsformen für das hämostyptisch wirksame Material haben den Vorteil, dass sich die funktionellen Gruppen aufgrund ihrer gleichnamigen Ladungen gegenseitig abstoßen, wodurch Zwischenräume entstehen. In diese Zwischenräume kann Wasser interkalieren. Dies wiederum erhöht die Saugkraft des hämostyptisch wirksamen Materials und damit des Hämostyptikums. Die erhöhte Saugkraft führt zu einer stärkeren Konzentration von Blutplättchen, wodurch eine beschleunigte Ausbildung eines natürlichen Fibrinnetzes und damit eine beschleunigte Blutstillung erzielbar ist.

In einer bevorzugten Ausführungsform besitzt das hämostyptisch wirksame Material eine Saugkraft, welches wenigstens dem achtfachen seines Eigengewichts entspricht.

Das hämostyptisch wirksame Material besitzt in einer weiteren Ausführungsform eine geringe Wasser- oder Blutlöslichkeit. Dadurch ist gewährleistet, dass das Hämostyptikum den Kontakt zu einem Behandlungssitus, insbesondere einer Wundoberfläche, nicht verliert und insbesondere nicht unter Verlust einer Barrierefunktion aus einem Behandlungssitus weggespült wird.

In einer weiteren Ausführungsform ist das hämostyptisch wirksame Material lediglich in zwei Dimensionen quellbar. Dadurch kann mit besonderem Vorteil eine Kompression von Organen, die in senkrechter Richtung zu einem Behandlungssitus angeordnet sind, vermieden werden. Dies ist besonders vorteilhaft bei der Behandlung von Gehirn- oder Herzbereichen, um die Manifestation von Nekrosen infolge einer Unterbindung der Blutversorgung funktionaler Bereiche zu vermeiden.

In einer besonders bevorzugten Ausführungsform liegt das hämostyptisch wirksame Material vernetzt, vorzugsweise chemisch vernetzt, vor. Hierdurch kann eine Unlöslichkeit des Hämostyptikums in Blut erzielt und eine Wegspülung des Hämostyptikums aus einem Behandlungssitus, beispielsweise infolge von überschüssigem Blut, vermieden werden. Obendrein kann eine Vernetzung, vorzugsweise chemische Vernetzung, des hämostyptisch wirksamen Materials mit besonderem Vorteil dazu beitragen, dass dieses entlang einer Behandlungsoberfläche wie beispielsweise Wundoberfläche, jedoch nicht senkrecht zu einer solchen Oberfläche quillt. Dadurch kann - wie bereits im vorherigen Absatz erwähnt - eine Kompression von gegenüberliegenden Strukturen, insbesondere Organen, vermieden werden, was insbesondere dann von Vorteil ist, wenn die Strukturen bzw. Organe dicht aneinander liegen.

Das hämostyptisch wirksame Material liegt in einer weitergehenden Ausführungsform mittels eines Vernetzungsagens chemisch vernetzt vor, wobei das Vernetzungsagens aus der Gruppe umfassend Glykolsäure, Milchsäure, Apfelsäure, Zitronensäure und Kombinationen davon ausgewählt ist. Bevorzugt handelt es sich bei dem Vernetzungsagens um Glykolsäure. Ein Beispiel für ein entsprechend vernetztes, hämostyptisch wirksames Material ist die im Folgenden noch genannte Croscarmellose bzw. das sogenannte Croscarmellose-Natrium (Natriumsalz einer mittels Glykolsäure quervernetzten Carboxymethylcellulose).

Das hämostyptisch wirksame Material ist in einer weiteren Ausführungsform ein Tablettensprengmittel. Überraschenderweise stellte sich insoweit heraus, dass Tablettensprengmittel Eigenschaften besitzen, die für die Erzielung einer Blutstillung günstig sind. So zeichnen sich Tablettensprengmittel in der Regel durch eine geringe Wasserlöslichkeit, eine geringe Gelbildungstendenz sowie durch eine hohe Kapillarität aus. Zudem besitzen Tablettensprengmittel in der Regel eine hervorragende Wasseraufnahmetendenz, wobei sich die Quellung bezüglich der Dimensionen (zwei- oder dreidimensional) beispielsweise durch eine Vernetzung einstellen bzw. kontrollieren lässt.

Das hämostyptisch wirksame Material ist vorzugsweise ausgewählt aus der Gruppe umfassend Proteine, Polysaccharide, insbesondere anionische Polysaccharide, Salze davon und Kombinationen davon.

In einer weitergehenden Ausführungsform ist das hämostyptisch wirksame Material ausgewählt aus der Gruppe umfassend Polyvinylpolypyrrolidon, Polyvinylpyrrolidon, Stärke, wasserquellende Stärke, modifizierte Stärke, Stärkeglykolat, Carboxymethylstärke, Gellan, Alginate, Dextrane, Chitin, Chitosan, Cellulose, mikrokristalline Cellulose, modifizierte Cellulose, Carboxymethylcellulose, Croscarmellose, Kollagen, Gelatine bzw. deren Hydrolysate, Agar, Albumin, Keratin, Seide, Kasein, Sojapeptid, Salze, insbesondere Alkalimetallsalze wie beispielsweise Natriumsalze, davon und Kombinationen davon.

Besonders bevorzugt ist das hämostyptisch wirksame Material ausgewählt aus der Gruppe umfassend Carboxymethylstärke, Distärkephosphat, acetylierte Distärkephosphat, Salze, insbesondere Alkalimetallsalze wie beispielsweise Natriumsalze, davon und Kombinationen davon.

Die oben genannte Carboxymethylstärke kann sich durch wenigstens einen der folgenden Spezifikationsparameter auszeichnen:

Einen Feuchtigskeitsverlust von maximal 7 %, einen Natriumanteil von 2.8 % bis 4.2 %, einen Substitutionsgrad von 0.21 bis 0.35, einen Natriumchloridanteil von maximal 7 %, einen Natriumglykolatanteil von maximal 2 %, einen pH-Wert von 5.5 bis 7.5, einen Eisenanteil von maximal 20 ppm sowie einen Schwermetallanteil von maximal 20 ppm.

Die oben erwähnte Distärkephosphat kann sich durch wenigstens einen der folgenden Spezifikationsparameter auszeichnen:

Einen Feuchtegehalt von maximal 7 % (Analysemethode: Infrarot-Feuchtemessgerät), einen pH-Wert von 5.5 bis 8.5 (Analysemethode: 5 %ig in destiliertem Wasser), eine Partikelgrößenverteilung von maximal 20 % > 500 $\mu$m (Analysemethode: Sieb 500 $\mu$m) sowie eine Viskosität von 2000 mPas bis 5000 mPas (Analysemethode: 5 %, nach Brookfield).

Das oben erwähnte acetylierte Distärkephosphat kann durch wenigstens einen der folgenden Spezifikationsparameter gekennzeichnet sein:

Einen Feuchtegehalt von maximal 7 % (Analysemethode: Infrarot-Feuchtemessgerät), einen pH-Wert von 6.0 bis 7.5 (Analysemethode: 5 %ig in destilliertem Wasser), eine Partikelgrößenverteilung von maximal 5 % > 200 $\mu$m und mindestens 25 % < 63 $\mu$m (Analysemethoden: Sieb 200 $\mu$m und Sieb 63 $\mu$m) sowie eine Viskosität von 3000 mPas bis 6500 mPas (Analysemethode: 5 %, nach Brookfield).

Das Hämostyptikum weist in einer weiteren Ausführungsform einen Anteil an dem hämostyptisch wirksamen Material von 90 Gew.-% bis 99.99 Gew.-%, insbesondere 95 Gew.-% bis 99.95 Gew.-%, bevorzugt 99 Gew.-% bis 99.9 Gew.-%, auf, bezogen auf das Gesamtgewicht des Hämostyptikums.

Das Visualisierungsmittel bewirkt in einer bevorzugten Ausführungsform bei Kontakt mit Blut eine von Blut unterscheidbare Färbung, insbesondere eine visuell erkennbare Färbung. Die Färbung kann im sichtbaren oder unsichtbaren Spektralbereich liegen. Beispielsweise kann das Visualisierungsmittel bei Kontakt mit Blut eine Schwarzfärbung im sichtbaren Spektralbereich oder eine Weißfärbung im Röntgenbild hervorrufen. Mit besonderem Vorteil lässt sich die Intensität einer Blutung anhand der Intensität einer Schwarzfärbung des Visualisierungsmittels und damit des erfindungsgemäßen Hämostyptikums erkennen, wodurch eine Dosierung des Hämostyptikums besser gesteuert werden kann.

Das Visualisierungsmittel bildet vorzugsweise mit Hämoglobin eine von Blut farblich unterscheidbare Verbindung, insbesondere Komplexverbindung.

Bei der Verbindung bzw. Komplexverbindung handelt es sich vorzugsweise um eine Anthrachinonverbindung bzw. um einen Anthrachinonkomplex. Mit anderen Worten handelt es sich bei dem Visualisierungsmittel vorzugsweise um einen mit Blut, insbesondere mit Hämoglobin, komplexbildenden Farbstoff. Beispiele für derartige Farbstoffe sind im folgenden Absatz genannt.

Das Visualisierungsmittel ist in einer weitergehenden Ausführungsform ein D & C Farbstoff, insbesondere ausgewählt aus der Gruppe umfassend D & C Grün Nr. 6 (1-4-bis{(4-Methylphenyl)amino)-9, 10-anthracendion), D & C Violet Nr. 2 (1,4-Dihydroxy-9,10-anthracendion) und Kombinationen davon.

In einer ergänzenden oder alternativen Ausführungsform ruft das Visualisierungsmittel bei Kontakt mit Blut eine durch diagnostische Methoden ermittel- bzw. nachweisbare Färbung auf. Beispielsweise kann es sich bei dem Visualisierungsmittel um ein Röntgen- oder Magnetresonanztomographie-Kontrastmittel (MRI-Kontrastmittel), insbesondere Bariumsulfat und/oder Gadoliniumsulfat, handeln.

Weiterhin kann das Hämostyptikum einen Anteil an dem Visualisierungsmittel von 0.01 Gew.-% bis 5 Gew.-%, insbesondere 0.05 Gew.-% bis 1 Gew.-%, bevorzugt 0.1 Gew.-% bis 0.5 Gew.-%, aufweisen, bezogen auf das Gesamtgewicht des Hämostyptikums.

Um die Benetzbarkeit des Hämostyptikums zu verbessern, kann es in einer weiteren Ausführungsform vorgesehen sein, dass das Hämostyptikum ein Benetzungsmittel aufweist.

Grundsätzlich kann es sich bei dem Benetzungsmittel um ein ionisches Tensid handeln. Geeignete ionische Tenside können beispielsweise aus der Gruppe umfassend Fettalkoholsulfate, Alkylsulfonate, Alkylbenzylsulfonate, Alkylcarboxylate und Kombinationen davon ausgewählt sein.

Erfindungsgemäß ist es indes bevorzugt, wenn das Hämostyptikum ferner ein nichtionisches Tensid aufweist.

Bei dem nichtionischen Tensid kann es sich insbesondere um ein Tensid handeln, welches als pharmazeutischer oder kosmetischer Hilfsstoff oder als Lebensmittelzusatzstoff zugelassen ist.

Geeignete nichtionische Tenside können aus der Gruppe umfassend Fettalkohole, ethoxylierte Fettalkohole, propoxylierte Fettalkohole, Blockcopolymere aus Ethylenoxid und Propylenoxid, Alkylphenolethoxylate, Alkylpolyglucoside, ethoxylierte Öle und/oder Fette, Alkanolamide, ethoxylierte Alkanolamide, ethoxylierte Fettsäuren, Glykole, ethoxylierte Glykole, Glycerinester, ethoxylierte Glycerinester, Sorbitanester, ethoxylierte Sorbitanester, alkylierte Kohlenhydratester, ethoxylierte Pentaerythritolester, Polyglycerolmonoester, Aminoxide und Kombinationen davon ausgewählt sein.

Das Hämostyptikum kann einen Anteil an dem Benetzungsmittel von 0.001 Gew.-% bis 9.999 Gew.-%, insbesondere 0.001 Gew.-% bis 4 Gew.-%, bevorzugt 0.001 Gew.-% bis 0.5 Gew.-%, aufweisen, bezogen auf das Gesamtgewicht des Hämostyptikums.

Das Hämostyptikum liegt in einer weiteren Ausführungsform in sprühbarer, insbesondere aerolisierbarer, Form vor.

Vorzugsweise ist das Hämostyptikum zur Verabreichung bzw. zum Austrag in Form eines Aerosols vorgesehen, welches Partikel des Hämostyptikums, insbesondere des hämostyptisch wirksamen Materials und/oder des Visualisierungsmittels, und ein Treibgas oder Treibgasgemisch enthält oder aus diesen Komponenten besteht. Bezüglich geeigneter Treibgase wird auf die im Folgenden noch genannten Treibgase Bezug genommen.

Das Hämostyptikum kann weiterhin Additive aufweisen, welche vorzugsweise ausgewählt sind aus der Gruppe umfassend antimikrobielle, insbesondere antibiotische, Wirkstoffe, beispielsweise Silberpartikel und/oder Polyhexamethylenbiguanid (PHMB), desinfizierende Wirkstoffe, entzündungshemmende Wirkstoffe, schmerzlindernde Wirkstoffe, geruchsbekämpfende Wirkstoffe und Kombinationen davon.

In einer weiteren Ausführungsform besteht das Hämostyptikum aus dem hämostyptisch wirksamen Material und dem Visualisierungsmittel. Gegebenenfalls kann das Hämostyptikum zusätzlich aus einem Benetzungsmittel und/oder weiteren Additiven bestehen. Insbesondere kann das Hämostyptikum in Form einer Mischung aus dem hämostyptisch wirksamen Material und dem Visualisierungsmittel sowie gegebenenfalls einem Benetzungsmittels und/oder weiteren Additiven bestehen. Bezüglich des Benetzungsmittels und der weiteren Additive wird vollständig auf die bisherige Beschreibung Bezug genommen.

Das Hämostyptikum kann in einer zweckmäßigen Ausführungsform in steriler Form vorliegen.

Das Hämostyptikum eignet sich grundsätzlich zur Blutstillung bei äußeren und/oder inneren, insbesondere parenchymatösen, Blutungen.

Das Hämostyptikum eignet sich weiterhin insbesondere zur Blutstillung von venösen und/oder arteriellen Blutungen, beispielsweise von arteriellen Blutungen in Folge von Nadelstichen oder in Folge von Anastomosenleckagen.

Ein bevorzugtes Anwendungsgebiet des Hämostyptikums betrifft die Behandlung von Kapillarblutungen.

Darüber hinaus eignet sich das erfindungsgemäße Hämostyptikum mit besonderem Vorteil zur Prophylaxe bzw. Vermeidung von (iatrogenen) Thrombosen, insbesondere (iatrogenen) Mikrothrombosen. Dies ist beispielsweise mittels einfachen Ausspülens des Hämostyptikums nach der Blutstillung realisierbar.

Ein zweiter Aspekt der Erfindung betrifft ein Kit. Das Kit umfasst das im Rahmen des ersten Erfindungsaspektes beschriebene Hämostyptikum und ein Treibgas oder Treibgasgemisch. Üblicherweise liegen das Hämostyptikum sowie das Treibgas bzw. Treibgasgemisch räumlich bzw. physikalisch voneinander getrennt vor. Beispielsweise können das Hämostyptikum und das Treibgas bzw. Treibgasgemisch in separaten Behältnissen bzw. Containern des Kits enthalten sein. Insbesondere können das Hämostyptikum und das Treibgas bzw. Treibgasgemisch in separaten Behältnissen bzw. Containern, insbesondere in separaten Kammern einer Zweikammer-Austragsvorrichtung, beispielsweise einer Zwillingsspritze, enthalten sein.

Bei dem Treibgas bzw. Treibgasgemisch handelt es sich in einer bevorzugten Ausführungsform um ein nicht brennbares bzw. nicht entzündliches Treibgas bzw. Treibgasgemisch.

In einer unter Umweltgesichtspunkten vorteilhaften Ausführungsform handelt es sich bei dem Treibgas bzw. Treibgasgemisch um ein klimafreundliches, insbesondere ozonschonendes, Treibgas bzw. Treibgasgemisch.

In einer zweckmäßigen Ausführungsform handelt es sich bei dem Treibgas bzw. Treibgasgemisch um ein in einer Vorlage, wie beispielsweise einem Kitbehältnis bzw. Kitcontainer, verflüssigbares Treibgas bzw. Treibgasgemisch.

Bevorzugt liegt das Treibgas bzw. Treibgasgemisch in verflüssigter Form vor. Mit anderen Worten ist es bevorzugt, wenn das Kit neben dem Hämostyptikum ein verflüssigtes Treibgas bzw. Treibgasgemisch umfasst.

Mit besonderem Vorteil ist das Treibgas bzw. Treibgasgemisch in der Lage, das Hämostyptikum ohne Verklumpung zu dispergieren.

Bei dem Treibgas bzw. Treibgasgemisch kann es sich insbesondere um ein Gas bzw. Gasgemisch handeln, welches das Hämostyptikum um nicht mehr als 10° Celsius abkühlt. Hierdurch kann beispielsweise vermieden werden, dass das Hämostyptikum bei Bildung von Kondenswasser an der Austrittsöffnung eines Steigrohres verklumpt.

In einer weiteren Ausführungsform weist das Treibgas einen Siedepunkt oberhalb von 15° Celsius oder eine Inversionstemperatur unterhalb von Raumtemperatur, bevorzugt unterhalb eines Temperaturbereiches von 20 °C bis 25 °C, auf.

Das Treibgas ist in bevorzugten Ausführungsformen ausgewählt aus der Gruppe umfassend perfluorierte Ether, Fluorkohlenwasserstoffe mit zwei bis fünf Kohlenstoffatomen, Tetrafluorethan, Hexafluorpropan, Heptafluorpropan, Decafluorbutan, Octafluorcyclobutan, Octafluortetrahydrofuran, Edelgase, Stickstoff, Sauerstoff, Kohlendioxid, Distickstoffoxid, Druckluft und Gemische davon.

Besonders bevorzugt ist das Treibgas ausgewählt aus der Gruppe umfassend Hexafluorpropan, Octafluorcyclobutan, Octafluortetrahydrofuran, Kohlendioxid und Gemische davon. Bei diesen Gasen handelt es sich vorteilhafterweise um kommerziell verfügbare, preiswerte, nicht entzündliche, klimaneutrale sowie in einer Vorlage verflüssigbare Gase.

Anstelle des im Rahmen des ersten Erfindungsaspektes beschriebenen Hämostyptikums kann das Kit auch räumlich bzw. physikalisch voneinander getrennt ein hämostyptisch wirksames Material und ein Visualisierungsmittel aufweisen. Mit anderen Worten kann das Kit somit räumlich voneinander getrennt ein hämostyptisch wirksames Material, ein Visualisierungsmittel sowie ein Treibgas oder Treibgasgemisch aufweisen.

Bezüglich weiterer Merkmale und Vorteile des im Rahmen des zweiten Erfindungsaspektes beschriebenen Kits, insbesondere des Hämostyptikums, des hämostyptisch wirksamen Materials sowie des Visualisierungsmittels, wird vollständig auf die im Rahmen des ersten Erfindungsaspektes gemachten Ausführungen Bezug genommen.

Ein dritter Erfindungsaspekt betrifft eine Austragsvorrichtung. Die Austragsvorrichtung umfasst das im Rahmen des ersten Erfindungsaspektes beschriebene Hämostyptikum und ein Treibgas oder Treibgasgemisch. Üblicherweise liegen das Hämostyptikum sowie das Treibgas bzw. Treibgasgemisch räumlich bzw. physikalisch voneinander getrennt vor. Beispielsweise können das Hämostyptikum und das Treibgas bzw. Treibgasgemisch in separaten Behältnissen bzw. Containern, insbesondere einer Zweikammer-Austragsvorrichtung, beispielsweise einer Zwillingsspritze, enthalten sein.

Die Austragsvorrichtung ist vorzugsweise als Sprühvorrichtung ausgestaltet.

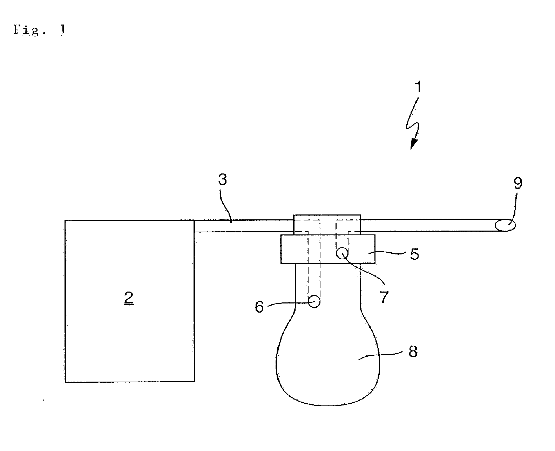

Bevorzugt umfasst die Austragsvorrichtung ein Behältnis zur Aufbewahrung des Hämostyptikums sowie ein Behältnis zur Aufbewahrung des Treibgases bzw. Treibgasgemisches. Vorzugsweise sind die beiden Behältnisse fluidleitend miteinander verbunden. Ein Beispiel für eine derartige Austragsvorrichtung ist schematisch in der Figur 1 dargestellt.

Bezüglich weiterer Merkmale und Vorteile der Austragsvorrichtung, insbesondere des Hämostyptikums und des Treibgases bzw. Treibgasgemisches, wird vollständig auf die im Rahmen des ersten und zweiten Erfindungsaspektes gemachten Ausführungen Bezug genommen.

Weitere Merkmale und Vorteile der Erfindung ergeben sich aus der nachfolgenden Beschreibung von bevorzugten Ausführungsformen anhand einer Figurenbeschreibung sowie von Ausführungsbeispielen. Dabei können einzelne Merkmale der Erfindung jeweils für sich alleine oder zu mehreren in Kombination miteinander verwirklicht sein. Die bevorzugten Ausführungsformen dienen lediglich der Erläuterung der vorliegenden Erfindung, die in keiner Weise darauf beschränkt sein soll.

**Figurenbeschreibung**

Figur 1 zeigt schematisch eine erfindungsgemäße Austragsvorrichtung 1, welche sich insbesondere zum Sprühaustrag des erfindungsgemäßen Hämostyptikums eignet.

Die Vorrichtung 1 umfasst ein Behältnis 2, in welchem ein Treibgas oder Treibgasgemisch erzeugt oder gelagert ist, und ein Behältnis 8, in welchem das zweckmäßigerweise in Form von Partikeln vorliegende Hämostyptikum gelagert ist, sowie eine Austragsspitze 9.

Die Behältnisse 2 und 8 sind zur Überleitung des Treibgases bzw. Treibgasgemisches von dem Behältnis 2 in das Behältnis 8 über eine Leitung 3 fluidleitend miteinander verbunden. Die Leitung 3 endet in dem Behältnis 8 vorzugsweise in Form eines Steigrohres 6. Das Steigrohr 6 kann dabei oberhalb der Partikel enden oder derart weit in das Behältnis 8 hineinragen, dass die Austrittsöffnung des Steigrohres 6 in die Partikel "eintaucht".

Das Behältnis 8 kann weiterhin über eine Verschlusseinrichtung 5 an der Austragsvorrichtung 1 befestigt sein. Die Befestigung kann auf einem Schraub- oder Schnappverschluss basieren. Beispielsweise kann die Befestigung über ein Außengewinde des Behältnisses 8 sowie ein hierzu passendes Innengewinde der Verschlusseinrichtung 5 erfolgen.

Mit besonderem Vorteil kann das Treibgas bzw. Treibgasgemisch, nachdem es die Leitung 3 passiert hat, über das Steigrohr 6 in das Behältnis 8 gelangen. Die Überleitung des Treibgases bzw. Treibgasgemisches in das Behältnis 8 kann beispielsweise mittels Ausübens von manuellem Druck auf das Behältnis 2, mittels Ausnutzen eines Überdrucks in dem Behältnis 2 bzw. mittels Pressluft geschehen. Durch die Überleitung des Treibgases bzw. Treibgasgemisches in das Behältnis 8 wird das darin gelagerte Hämostyptikum aufgewirbelt und kann als Aerosol über eine in der Verschlusseinrichtung 5 angeordnete Öffnung 7, die eine fluidleitende Verbindung zur Austragsspitze 9 herstellt, das Behältnis 8 in Richtung der Austragsspitze 9 verlassen.

Der Austrag des Aerosols erfolgt schlussendlich über die Austragsspitze 9, welche hierfür kanülenförmig oder schlitzförmig ausgestaltet sein kann.

Das Hämostyptikum liegt vorzugsweise in Form einer Mischung vor, welche das hämostyptisch wirksame Material sowie das Visualisierungsmittel enthält.

**Ausführungsbeispiele**

Beispiel 1:

In einer 35ml Aerosoldose wurden 5.0 g ultrafein gemahlenes hämostyptisches Pulver einer Korngröße von $D_{90}$ = 30 $\mu$m (Kollagenpulver, Ultramyl, Vivastar P 5000, Spezialstärke 6039G, Eskugel GP AC F7, Polyplasdone XL-10, Avicel) vorgelegt und anschließend über eine Druckdose mit 20 g Hexafluorpropan als Treibmittel beaufschlagt. Bei einem anschließenden Sprühversuch konnte das gesamte Pulver aus der Druckdose über ein Metallsprühventil entleert werden.

Beispiel 2:

In kleinen Zentrifugengläsern wurden etwa 100 mg Substanz (siehe die in Tabelle 1 genannten Substanzen) bis auf 1 mg genau eingewogen, mit etwa 4 ml Plasmaexpander (Gelafundin® von B. Braun) ebenfalls auf 1 mg genau eingewogen, überschichtet und anschließend für 5 Minuten auf dem Vortexrührer vermischt. Es waren keine trockenen Reste des Pulvers im Zentrifugenglas zu erkennen. Anschließend wurde in einer Ultrazentrifuge der Firma Hettich (Rotana 460 R) bei 11500 U/min für 10 Minuten bei 23°C zentrifugiert.

Die überstehende Lösung und der zurückgebliebene gequollene Feststoff wurden ausgewogen und daraus die Saugkapazität des Pulvers nach folgender Formel bestimmt:

$$\text{Saugkapazität} = [m(\text{gequollenes Pulver}) - m(\text{trockenes Pulver}) / m(\text{trockenes Pulver})] \times 100\,\%.$$

Die hiernach bestimmten Saugkapazitäten sind in untenstehender Tabelle 1 ausgeführt.

Tabelle 1: Bestimmung der Saugkapazität verschiedener Substanzen

| Substanz | Saugkapazität |
|---|---|
| Ultramyl | 13.1 |

(fortgesetzt)

| Substanz | Saugkapazität |
|---|---|
| Vivastar P5000 | 12.9 |
| Spezialstärke 6039G | 8.4 |
| Eskugel GP AC 7F | 8.6 |
| Ac-Di-Sol | 7.9 |
| Kollagenpulver | 5.3 |
| Polyplasdone XL-1 0 | 4.5 |

Beispiel 3:

In kleinen Petrischalen (Durchmesser 35 mm) wurden etwa 100 mg Substanz aus untenstehender Tabelle 2 auf 1 mg genau eingewogen, vorgelegt und mit 1.0 mL nicht heparinisiertem, frischem auf 0°C vorgekühltem Humanblut überschichtet. Mit einem Polyethylenspatel wurden das Blut und die Substanz gleichmäßig vermischt. Anschließend wurde ein neuer Polyethylenspatel in regelmäßigen Zeitabständen in das Blut eingetaucht, umgerührt und vorsichtig aus der Lösung gezogen, bis sich dünne Fibrinfäden erkennen ließen. Die Zeit bis zur Bildung dieser Fibrinfäden wurde gestoppt.

Mit zunehmender Zeit bildeten sich Fibrinfäden in der folgenden, in Tabelle 2 wiedergegebenen Reihenfolge aus.

Tabelle 2: Zeit in Minuten bis zur Ausbildung von Fibrinfäden

| Substanz | Zeit (min) bis zur Bildung von Fibrinsträngen |
|---|---|
| Stahl (Positivkontrolle) | 1 |
| Ac-Di-Sol | 8 |
| Specialstärke 6039G | 10 |
| Eskugel GP AC F7 | 14 |
| Kollagen | 15 |
| Ultramyl | 16 |
| Avicel | 19 |
| Polyplasdone XL-1 0 | 22 |
| Blank (Negativkontrolle) | 50 |

Beispiel 4:

Zu 1.00 g eines Carboxymethylcellulose-Sols wurden 1 mg eines D & C Farbstoffes zugesetzt und in einer Reibschale mit Pistill für 10 Minuten gründlich verrieben. 100 mg der Mischung wurden danach in eine Petrischale (Durchmesser 35 mm) gefüllt und mit 1.0 ml Humanblut überschichtet. Man erhielt im Bereich des Pulvers eine deutliche Farbschattierung, die innerhalb des Blutes den Verbleib des Pulvers anzeigte.

Es wurden zwei Farbstoffe eingesetzt: D & C Grün Nr. 6, namentlich 1-4-bis{(4-Methylphenyl)amino}-9,10-anthracendion (74.3206) und D & C Violett Nr. 2, namentlich 1,4-Dihydroxy-9,10-anthracendion (74.3602). Beide Farbstoffe bildeten mit dem Blutfarbstoff Hämoglobin dunkelfarbige Komplexe.

Beispiel 5:

In einem Schafstierversuch wurde nach einer medianen Laparotomie auf einem Leberlappen ein 2 cm langer und 5 cm tiefer Skalpellschnitt in das Parenchymgewebe vorgenommen. Unmittelbar anschließend wurden über eine Aerosolspraydose etwa 3 g eines Hämostyptikums (siehe untenstehende Tabelle 3) auf die Wunde appliziert und mit einer Baumwollgauze die Wunde (und damit die verletzten Gefäße) für eine Dauer von 140 Sekunden komprimiert. Nach vorsichtiger Entfernung der Gauze und Spülung der Oberfläche mit physiologischer Kochsalzlösung wurde der Zustand der Blutstillung (+ bedeutet: Blutung wurde nach 140 Sekunden vollständig gestoppt, - bedeutet: Blutung wurde nach 140 Sekunden nicht gestoppt) beurteilt (siehe untenstehende Tabelle 3). Bei erfolgreicher Blutstillung bildete sich ein Blutpfropf zusammen mit dem Hämostyptikum.

Tabelle 3: Erfolg der Blutstillung mit verschiedenen Substanzen

| Hämostyptikum | Blutstillung |
|---|---|
| Ultramyl | + |
| Vivastar P 5000 | - |
| HämoCer™ | + |
| Avicel | + |
| Kollagen | + |
| Ac-Di-Sol | + |

Bei dem Versuch zeigte sich, dass insbesondere eine möglichst kurze Benetzungszeit, vorzugsweise in Kombination mit einer guten Saugkraft, des Hämostyptikums zu zufriedenstellenden Ergebnissen führte

An dieser Stelle sollen die Vorteile des erfindungsgemäßen Hämostyptikums noch einmal wie folgt zusammengefasst werden:

Das Hämostyptikum kann in besonders vorteilhafter Weise als Aerosol mittels einer Sprayvorrichtung, beispielsweise Spraydose, appliziert werden. Es eignet sich insbesondere zur Blutstillung in einem eng begrenzten Operationsfeld wie beispielsweise laparoskopischen Herzoperationen. Das wenigstens eine Visualisierungsmittel lässt insbesondere durch Hervorrufung einer von Blut unterscheidbaren Färbung, beispielsweise Schwarzfärbung, bei Kontakt mit Blut eine Beurteilung der Hämostasewirkung zu, da der Blutzufluss zu überschüssigem Hämostyptikum im Operationsfeld sichtbar wird.

**Patentansprüche**

1. Hämostyptikum, umfassend ein hämostyptisch wirksames Material und ein Visualisierungsmittel.

2. Hämpostyptikum nach Anspruch 1, **dadurch gekennzeichnet, dass** das Hämostyptikum in Form einer das hämostyptisch wirksame Material und das Visualisierungsmittel enthaltenden Mischung vorliegt.

3. Hämpostyptikum nach Anspruch 1 oder 2, **dadurch gekennzeichnet, dass** das Hämostyptikum in partikulärer Form, vorzugsweise in Form eines Pulvers, vorliegt.

4. Hämpostyptikum nach einem der vorhergehenden Ansprüche, **dadurch gekennzeichnet, dass** das Hämpostyptikum eine Partikelgröße < 200 μm, insbesondere von 1 μm bis 60 μm, aufweist.

5. Hämostyptikum nach einem der vorhergehenden Ansprüche, **dadurch gekennzeichnet, dass** das hämostyptisch wirksame Material ein mucoadhäsives Material ist.

6. Hämostyptikum nach einem der vorhergehenden Ansprüche, **dadurch gekennzeichnet, dass** das hämostyptisch wirksame Material funktionelle Gruppen aufweist, die ausgewählt sind aus der Gruppe umfassend Carboxylgruppen, Sulfatgruppen, Phosphatgruppen und Kombinationen davon.

7. Hämostyptikum nach einem der vorhergehenden Ansprüche, **dadurch gekennzeichnet, dass** das hämostyptisch wirksame Material ein vernetztes, vorzugsweise chemisch vernetztes, Material ist.

8. Hämostyptikum nach einem der vorhergehenden Ansprüche, **dadurch gekennzeichnet, dass** das hämostyptisch wirksame Material ein Tablettensprengmittel ist.

9. Hämostyptikum nach einem der vorhergehenden Ansprüche, **dadurch gekennzeichnet, dass** das hämostyptisch wirksame Material ausgewählt ist aus der Gruppe umfassend Polyvinylpolypyrrolidon, wasserquellende Stärke, modifizierte Stärke, Alginate, Dextrane, Carboxymethylstärke, Cellulose, mikrokristalline Cellulose, modifizierte Cellulose, Carboxymethylcellulose, Croscarmellose, Kollagen, Salze, insbesondere Natriumsalze, davon und Kombinationen davon.

**10.** Hämostyptikum nach einem der vorhergehenden Ansprüche, **dadurch gekennzeichnet, dass** das Visualisierungsmittel bei Kontakt mit Blut eine von Blut unterscheidbare Färbung, insbesondere Schwarzfärbung, hervorruft.

**11.** Hämostyptikum nach einem der vorhergehenden Ansprüche, **dadurch gekennzeichnet, dass** das Visualisierungsmittel in der Lage ist, einen Kontrast gegenüber einem das Hämostyptikum umgebenden Blut herzustellen.

**12.** Hämostyptikum nach einem der vorhergehenden Ansprüche, **dadurch gekennzeichnet, dass** es sich bei dem Visualisierungsmittel um einen D & C Farbstoff, insbesondere ausgewählt aus der Gruppe umfassend D & C Grün Nr. 6 (1-4-bis{(4-Methylphenyl)amino}-9,10-anthracendion), D & C Violett Nr. 2 (1,4-Dihydroxy-9,10-anthracendion) und Kombinationen davon, und/oder um ein Röntgen- oder Magnetresonanztomographie-Kontrastmittel, insbesondere ausgewählt aus der Gruppe umfassend Bariumsulfat, Gadoliniumsulfat und Kombinationen davon, handelt.

**13.** Hämostyptikum nach einem der vorhergehenden Ansprüche, **dadurch gekennzeichnet, dass** das Hämostyptikum ferner ein Benetzungsmittel, vorzugsweise in Form eines nichtionischen Tensids, aufweist.

**14.** Hämostyptikum nach einem der vorhergehenden Ansprüche, **dadurch gekennzeichnet, dass** das Hämostyptikum in sprühbarer, insbesondere aerolisierbarer, Form vorliegt.

**15.** Kit, umfassend räumlich voneinander getrennt ein Hämostyptikum nach einem der vorhergehenden Ansprüche und ein Treibgas.

**16.** Austragsvorrichtung, vorzugsweise Sprühvorrichtung, umfassend räumlich voneinander getrennt ein Hämostyptikum nach einem der Ansprüche 1 bis 14 und ein vorzugsweise nicht entzündliches, klimafreundliches und insbesondere verflüssigtes Treibgas.

Fig. 1

**Europäisches Patentamt**
**European Patent Office**
**Office européen des brevets**

**EUROPÄISCHER RECHERCHENBERICHT**

Nummer der Anmeldung

EP 14 17 2496

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2007/087061 A1 (DRAKE JAMES F [US] ET AL) 19. April 2007 (2007-04-19)<br>* Absatz [0002] *<br>* Absatz [0018] - Absatz [0019] *<br>* Absatz [0035] *<br>* Absatz [0036] *<br>* Absatz [0047] *<br>* Absatz [0050] *<br>* Absatz [0069] *<br>----- | 1-9,11, 14-16 | INV.<br>A61L26/00<br>A61L24/00<br>A61L24/06<br>A61L24/08<br>A61L24/10 |
| X | US 2012/041479 A1 (BASORE JR BOB O [US] ET AL) 16. Februar 2012 (2012-02-16)<br>* Absatz [0002] *<br>* Absatz [0008] *<br>* Absatz [0018] *<br>* Absatz [0197] *<br>* Absatz [0199] *<br>* Absatz [0219] *<br>* Absatz [0240] *<br>* Absatz [0248] *<br>* Absatz [0268] *<br>* Absatz [0271] *<br>----- | 1-3,5-16 | |
| X | US 2008/032934 A1 (ELLIS-BEHNKE RUTLEDGE [US] ET AL) 7. Februar 2008 (2008-02-07)<br>* Absatz [0008] - Absatz [0009] *<br>* Absatz [0014] - Absatz [0015] *<br>* Absatz [0086] *<br>* Absatz [0099] - Absatz [0100] *<br>* Absatz [0113] *<br>* Absatz [0133] *<br>* Absatz [0121] - Absatz [0122] *<br>* Absatz [0125] *<br>-----<br>-/-- | 1-3,5-9, 12-16 | RECHERCHIERTE SACHGEBIETE (IPC)<br>A61L |

Der vorliegende Recherchenbericht wurde für alle Patentansprüche erstellt

| Recherchenort | Abschlußdatum der Recherche | Prüfer |
|---|---|---|
| Den Haag | 24. Oktober 2014 | Zalfen, Alina |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
........................................................
& : Mitglied der gleichen Patentfamilie, übereinstimmendes Dokument

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 14 17 2496

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2012/123728 A2 (MEDTRADE PRODUCTS LTD; HARDY CRAIG [GB]; EASON GUY [GB]; HOGGARTH ANDR) 20. September 2012 (2012-09-20)<br>* Seite 2, Zeilen 13-14 *<br>* Seite 3, Zeile 18 - Seite 4, Zeile 2 *<br>* Seite 4, Zeilen 9-12 *<br>* Seite 6, Zeile 19 - Seite 7, Zeile 1 *<br>* Seite 8, Zeilen 12-21 *<br>* Seite 9, Zeilen 5-12 *<br>* Seite 11, Zeilen 3-4 *<br>----- | 1-3,5-13 | |
| X | US 2011/034412 A1 (YATABE TERUYUKI [JP] ET AL) 10. Februar 2011 (2011-02-10)<br><br>* Absatz [0002] *<br>* Absatz [0010] *<br>* Absatz [0029] *<br>* Absatz [0049] *<br>* Absatz [0073] *<br>* Absatz [0100] - Absatz [0101] *<br>* Absatz [0105] *<br>* Absatz [0106] *<br>----- | 1-3,<br>5-11,<br>13-16 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 24. Oktober 2014 | Zalfen, Alina |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**   EP 14 17 2496

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-10-2014

| Im Recherchenbericht angeführtes Patentdokument | Datum der Veröffentlichung | Mitglied(er) der Patentfamilie | Datum der Veröffentlichung |
|---|---|---|---|
| US 2007087061 A1 | 19-04-2007 | US 2007087061 A1<br>WO 2008048461 A2 | 19-04-2007<br>24-04-2008 |
| US 2012041479 A1 | 16-02-2012 | US 2012041479 A1<br>WO 2010129258 A2 | 16-02-2012<br>11-11-2010 |
| US 2008032934 A1 | 07-02-2008 | CA 2722465 A1<br>EP 2150268 A1<br>EP 2581097 A1<br>JP 2010526779 A<br>US 2008032934 A1<br>WO 2008134544 A1 | 06-11-2008<br>10-02-2010<br>17-04-2013<br>05-08-2010<br>07-02-2008<br>06-11-2008 |
| WO 2012123728 A2 | 20-09-2012 | CA 2829305 A1<br>EP 2683345 A2<br>GB 2488915 A<br>US 2014105950 A1<br>WO 2012123728 A2 | 20-09-2012<br>15-01-2014<br>12-09-2012<br>17-04-2014<br>20-09-2012 |
| US 2011034412 A1 | 10-02-2011 | CN 101998868 A<br>EP 2269665 A1<br>JP 5527898 B2<br>US 2011034412 A1<br>WO 2009133763 A1 | 30-03-2011<br>05-01-2011<br>25-06-2014<br>10-02-2011<br>05-11-2009 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 101497670 A
- WO 2011031457 A2
- WO 2009091549 A1
- WO 2005002510 A2
- EP 2233157 A1
- WO 9833479 A1
- EP 1025868 B1
- US 5676964 A
- CN 1269376 A

**In der Beschreibung aufgeführte Nicht-Patentliteratur**

- **THOMAS MEZGER.** Das Rheologie-Handbuch: Für Anwender von Rotations- und Oszillations-Rheometern. Curt R. Vincentz Verlag, 2010, 441